# EUROPEAN PATENT SPECIFICATION

(11) **EP 0 760 709 B1**
(45) Date of publication and mention of the grant of the patent: **06.05.1999**
(21) Application number: 95922472.6
(22) Date of filing: 22.05.1995
(51) Int. Cl.: B01J 23/28, C07D 213/84

(54) **CATALYTIC COMPOSITION FOR THE OXIDATIVE AMMONOLYSIS OF ALKYLPYRIDINES**
KATALYTISCHE ZUSAMMENSETZUNG ZUR AMMOXYDIERUNG VON ALKYLPYRIDINEN
COMPOSITION CATALYTIQUE UTILISEE POUR EFFECTUER L'AMMONOLYSE OXYDATIVE D'ALKYLPYRIDINES

(30) Priority: 23.05.1994 KZ 94056194; 11.08.1994 WO PCT/EP94/02676
(43) Date of publication of application: 12.03.1997
(73) Proprietor: LONZA LTD., CH-3945 Gampel (CH); INSTITUTE OF CHEMICAL SCIENCE OF THE NATIONAL ACADEMY OF SCIENCE OF THE REPUBLIC KAZAKSTAN, Almaty (KZ)
(72) Inventor: SEMBAEV, Dauren Chamitovitch, Almaty, 480002 (KZ); IVANOVSKAYA, Faina Alekseevna, Almaty, 480091 (KZ); GUSEINOV, Ernest Muslimovitch, Moscow, 117330 (RU); CHUCK, Roderick, John, CH-3902 Brig-Glis (CH)
(74) Representative: Weinhold, Peter, Dr.
(86) International application number: EP9501945
(87) International publication number: WO9532055

(56) References cited:
- EP-A- 0 290 996
- EP-A- 0 339 680
- EP-A- 0 514 682
- FR-A- 2 264 023
- US-A- 3 799 888
- US-A- 3 970 657
- US-A- 5 032 253

## Description

This invention relates to catalytic compositions, their use in the oxidative ammonolysis of alkylpyridines and to a process for the production of cyanopyridines.

Preferably the catalytic compositions are used for the oxidative ammonolysis of 3-methylpyridine and 2-methyl-5-ethylpyridine to the corresponding 3-cyanopyridine.

3-Cyanopyridine is an intermediate for nicotinic acid or nicotinic acid amide which are essential vitamins of the B-complex.

The oxidative ammonolysis of alkylpyridines is well known in the art. A great variety of catalyst systems have been disclosed but so far no process is known which can adequately satisfy the needs of a commercial process on a technical scale.

Reference is made to the USSR inventors certificate No. 891 142, wherein a catalyst for the ammonolysis of alkylpyridines consisting of the oxides of vanadium, tin and titanium is described. The maximum yield achieved for the conversion of for (e.g.) instance 2-methyl-5-ethylpyridine is 63%.
The main drawback of this catalytic composition therefore is its low activity and selectivity.

From Swiss patent 595 350, it is further known that 2-methyl-5-ethyl pyridine can be converted to 3-cyanopyridine over a supported mixed oxide catalyst composed of oxides of vanadium, zirconium or titanium and optionally of tungsten.
The yields obtained with this catalyst range between 60% and 75%. This catalyst is also not satisfactory due to its low selectivity and activity. A further drawback is the rather complicated procedure for producing this supported catalyst.
EP-A-0 339 680 describes a catalyst comprising V₂O₅, Mo oxide and Cr and B oxides and US-A-3799888 and describes a catalyst comprising V₂O₅ and TiO₂ both of which are used for the oxidative ammonolysis of 3-methylpyridine and 2-methyl-5-ethylpyridine.

The object of the present invention is to provide catalytic compositions with further improved catalytic activity and performance and therefore to provide an improved process for oxidative ammonolysis of alkylpyridines especially with respect to selectivity and yield.

The catalytic composition of the present invention according to claim 1 consists of the oxides of vanadium, zirconium, titanium and molybdenum having a molar ratio of V₂O₅ to TiO₂ to ZrO₂ from 1:1:2 to 1:12:25 and a MoO₃ content of 0.54 to 2.6 weight percent relating to V₂O₅.

A preferred catalytic composition has a molar ratio of V₂O₅ to TiO₂ to ZrO₂ from 1:3:4 to 1:8:16 and a MoO₃ content of 0.54 to 1.15 weight percent relating to V₂O₅.
In order to prepare the catalytic composition one can use the respective oxides itself but it is also possible to use precursor compounds which are later converted into the oxides. Such precursor compounds are for example: vanadiumoxide, the ammoniummetavanadate; zirconiumoxide, the zirconylchloride; titaniumoxide, the metatitaniumacid; and for the molybdenumoxide, the ammoniummolybdate.

The preparation of the catalytic composition can as a rule be accomplished by mixing the compounds in a suitable milling device, granulating or tabletting the mixture and finally drying the granules or tablets at a temperature of about 100°C to 120°C in a stream of air. The catalyst undergoes wiht advantage a subsequent thermal treatment at temperatures up to 650°C.
The ready prepared catalyst can then be charged into the reactor, wherein after an activation phase under reaction conditions, it is able to demonstrate its properties with respect both to high activity and selectivity at high loadings of the alkylpyridine and also with respect to long service life.
The catalytic composition of the present invention is especially suitable for the oxidative ammonolysis of alkylpyridines in presence of ammonia, an oxygen containing gas and if necessary water vapour. Preferred application of the catalytic composition is the conversion of 3-methylpyridine or 2-methyl-5-ethylpyridine to 3-cyanopyridine.
The following process conditions have been proved to be suitable.

Air is generally used as the oxygen-containing gas. Thus air offers the advantage that oxygen is already diluted with inert components. The partial pressure of oxygen can be advantageously regulated by further dilution with a suitable inert gas, for example nitrogen, or by recycling part or most of the oxygen-lean exhaust gases.

Two advantages over the processes from the known state of the art is offered in the conversion of 3-methylpyridine, which according to the invention not only needs no addition of water vapour, but also uses a practically stoichiometric quantity or a small molar excess of ammonia.

The gaseous feed of the reactants in the case of the oxidative ammonolysis of 3-methylpyridine is accordingly composed of a molar ratio of 3-methylpyridine to ammonia to air (calculated on the basis of oxygen) from 1:1:1.5 to 1:8.5:60.

A preferred gaseous feed is composed of a molar ratio of 3-methylpyridine to ammonia to air (calculated on the basis of oxygen) from 1:1:2 to 1:4:60

The gaseous feed of the reactants in the case of the oxidative ammonolysis of 2-methyl-5-ethylpyridine is composed of a molar ratio of 2-methyl-5-ethylpyridine to ammonia to air (calculated to O₂) and to water vapour from 1:20:20:60 to 1:60:70:330.
The temperature in the reaction zone of the catalyst bed ranges expediently between 280°C and 400°C and preferably between 310°C and 380°C.

The characteristics of the catalytic composition also with respect to life-time allow the process to be run continuously on a large scale-basis.

The maximum yield achieved with 3-cyanopyridine upon feeding up to 150 g per litre per hour of catalyst of 3-methylpyridine reaches 99%, and with 2-methyl-5-ethylpyridine up to 120 g per litre per hour of catalyst reaches 85%.

### Examples:

### Example 1

36.4 g of vanadiumpentoxide, 48.0 g of titaniumdioxide, 197.2 g of zirconiumdioxide and 0.42 g of molybdenumtrioxide in the molar ratio V₂O₅:TiO₂:ZrO₂ = 1:3:8 and 1.15 wt.% MoO₃ based on vanadiumpentoxide, were ground and mixed in a ball mill. The mixture was moulded into granules of 5 x 5 mm and thermally treated at a temperature of 100 - 120°C for 6 hours in a flow of air. The obtained catalyst in the quantitiy of 60 cm³ (82 g) was loaded into a tube reactor made of stainless steel (internal diameter 20 mm, length 1000 mm). A mixture of the reagents, consisting of 2-methyl-5-ethylpyridine, air, ammonia, water vapour was passed through the catalyst layer at a temperature of 340°C. The feeding rate (gram per 1 litre catalyst per 1 hour = gl⁻¹h⁻¹) was: 2-methyl-5-ethylpyridine - 72 gl⁻¹h⁻¹, air - 1500 litres, ammonia - 228 gl⁻¹h⁻¹ and water - 583.3 gl⁻¹h⁻¹ corresponding to a molar ratio of 2-methyl-5-ethylpyridine to oxygen to ammonia to water of 1:47:45:108. Accordingly 21.6 g of 2-methyl-5-ethylpyridine was fed over 10 hours. The conversion was complete. 15.0 g of 3-cyanopyridine was obtained corresponding to a yield of 80.5% from theory. The output of 3-cyanopyridine was 49.8 gl⁻¹h⁻¹.

### Example 2

A catalyst as described in example 1 was used. A mixture consisting of 3-methylpyridine, air and ammonia was passed through the catalyst at a temperature of 330°C. The feeding rate (gram per 1 litre catalyst per 1 hour = gl⁻¹h⁻¹) was: 3-methylpyridine - 84 gl⁻¹h⁻¹, air - 2000 litres, ammonia - 9.92 gl⁻¹h⁻¹ corresponding to a molar ratio of 3-methylpyridine: O₂ : NH₃ = 1:40:1.3. Accordingly 25.5 g of 3-methylpyridine was fed over 10 hours. The conversion was complete. 26.8 g of 3-cyanopyridine was obtained corresponding to a yield of 95.0% mol from theory. The output of 3-cyanopyridine was 89.2 gl⁻¹h⁻¹.

### Example 3

A catalyst was prepared from 36.4 g of vanadiumpentoxide, 64.0 g of titanium dioxide, 98.6 g of zirconium dioxide and 0.2 g of MoO₃ in the molar ratio V₂O₅:TiO₂:ZrO₂ = 1:4:4 and 0.54% wt.% MoO₃ based on V₂O₅.
The catalyst was prepared by the method described in the example 1. The gaseous feed consisting of 2-methyl-5-ethylpyridine air, ammonia and water vapour was passed through the catalyst bed (60 cm³) at a temperature of 320°C. The feeding rate (gram per 1 litre catalyst per 1 hour = gl⁻¹h⁻¹) was: 2-methyl-5-ethylpyridine - 72 gl⁻¹h⁻¹, air - 1500 litres, ammonia - 228 gl⁻¹h⁻¹, water 700 gl⁻¹h⁻¹ corresponding to a molar ratio of 2-methyl-5-ethylpyridine : O₂ : NH₃ : H₂O of 1:47:45:130. Accordingly 21.6 g of 2-methyl-5-ethylpyridine was fed over 10 hours. The conversion was complete. 15.3 g 3-cyanopyridine was obtained corresponding to a yield of 82.2% based on the feed of 2-methyl-5-ethylpyridine. The output of 3-cyanopyridine was 50.8 gl⁻¹h⁻¹.

### Example 4

A catalyst with a molar ratio of V₂O₅:TiO₂:ZrO₂ = 1:4:4 and 0.90 wt.% of MoO₃ based on V₂O₅ was prepared according to example 1. A mixture consisting of 3-methylpyridine, air, ammonia was passed through the catalyst at a temperature of 330°C. The feeding rate (gram per 1 litre catalyst per 1 hour = gl⁻¹h⁻¹) was: 3-methylpyridine - 84 gl⁻¹h⁻¹, air - 2000 litres, ammonia - 9.92 gl⁻¹h⁻¹ corresponding to a molar ratio of 3-methylpyridine: O₂ : NH₃ of 1:40:1.3. Accordingly 25.2 g of 3-methylpyridine was fed over 10 hours. The conversion was complete. 27.3 g 3-cyanopyridine was obtained corresponding to a yield of 97.9% from theory. The output of 3-cyanopyridine was 91.0 gl⁻¹h⁻¹.

### Example 5

A catalyst with a molar ratio of V₂O₅:TiO₂:ZrO₂ = 1:4:8 and 0.98 wt.% of MoO₃ based on V₂O₅ was prepared according to example 1. A mixture consisting of 2-methyl-5-ethylpyridine, air, ammonia and water vapour was passed through the catalyst at a temperature of 320°C. The feeding rate (gram per 1 litre catalyst per 1 hour = gl⁻¹h⁻¹) was: 2-methyl-5-ethylpyridine - 72 gl⁻¹h⁻¹, air - 1500 litres, ammonia - 228 gl⁻¹h⁻¹ and water - 700 gl⁻¹h⁻¹ corresponding to a molar ratio of 2-methyl-5-ethylpyridine: O₂ : NH₃ : H₂O of 1:47:45:130. Accordingly 21.6 g of 2-methyl-5-ethylpyridine was fed over 10 hours. The conversion was complete. 15.4 g of 3-cyanopyridine was obtained corrresponding to a yield of 83% based on the feed of 2-methyl-5-ethylpyridine. The output of 3-cyanopyridine was 51.3 gl⁻¹h⁻¹.

### Example 6

A catalyst with a molar ratio V₂O₅:TiO₂:ZrO₂ = 1:4:8 and 1.15 wt% of MoO₃ based on V₂O₅ was prepared according to example 1. A mixture consisting of 3-methylpyridine, air, ammonia was passed through the catalyst at a temperature of 325°C. The feeding rate (gram per 1 litre catalyst per 1 hour = gl⁻¹h⁻¹) was: 3-methylpyridine - 168 gl⁻¹h⁻¹, air - 2000 litres, ammonia - 22.8 gl⁻¹h⁻¹ corresponding to a ratio of 3-methylpyridine : O₂ : NH₃ of 1:40:1.5. Accordingly 50.4 g of 3-methylpyridine was fed over 10 hours. The conversion was complete. 55.8 g of 3-cyanopyridine was obtained corresponding to a yield of 99.0% from theory. The output of 3-cyanopyridine was 186 gl⁻¹h⁻¹.

### Example 7

A catalyst with the molar ratio V₂O₅:TiO₂:ZrO₂ = 1:4:8 and 1.15 wt% of MoO₃ based on V₂O₅ was prepared according to example 1. A mixture consisting of 3-methylpyridine, air, ammonia was passed through the catalyst at a temperature of 350°C. The feeding rate (gram per 1 litre catalyst per 1 hour = gl⁻¹h⁻¹) was 3-methylpyridine - 218 gl⁻¹h⁻¹, air - 2000 litres, ammonia - 30.35 gl⁻¹h⁻¹ corresponding to a molar ratio of 3-methylpyridine : O₂ : NH₃ of 1:16:1.5. Accordingly 65.5 g of 3-methylpyridine was fed over 10 hours. The conversion was complete. 75.2 g of 3-cyanopyridine was obtained corresponding to a yield of 99.0% from theory. The output of 3-cyanopyridine was 241.7 gl⁻¹h⁻¹.

### Example 8

1.167 kg of vanadiumpentoxide, 2.512 kg of titaniumdioxide as metatitanic acid, 6.322 kg of zirconiumdioxide and 12.4 g of ammoniumparamolybdate (molybdic acid) in the molar ratio V₂O₅:TiO₂:ZrO₂ = 1:4:8 and 1.05% (NH₄)₆ Mo₇O₂₄.4H₂O based on vanadiumpentoxide, were kneaded in a double-arm kneader and ground and mixed in a ball mill. The mixture was formed into granules of approximately 3 x 3 mm and thermally treated at a temperature of 100-120°C for 6 hours. A quantity of the obtained catalyst (1 liter, 1.50 kg) was loaded into a tube reactor made of stainless steel (internal diameter 21 mm, length 3 meters). A mixture of reagents, consisting of 3-methylpyridine, air, nitrogen and ammonia, was passed through the catalyst at a temperature of 340°C. The feeding rate (gram per 1 liter catalyst per hour = gl⁻¹h⁻¹) was: 3-methylpyridine 80 gl⁻¹h⁻¹, air 200 liter.h⁻¹, nitrogen 1200 liter.h⁻¹, ammonia 37.5 gl⁻¹h⁻¹ corresponding to a molar ratio of 3-methylpyridine to ammonia to oxygen of 1 : 2.6 : 2.2. Accordingly 1920 g 3-methylpyridine was fed over 24 hours. The conversion was 99%. 1910 g of 3-cyanopyridine were obtained corresponding to a yield of 89%. The output of 3-cyanopyridine was 79.6 gl⁻¹h⁻¹

### Example 9

A quantity of the obtained catalyst from example 8 (985 cm³, 1.46 kg) was loaded into a tube reactor made of stainless steel (internal diameter 21 mm, length 3 meters). A mixture of reagents, consisting of 3-methylpyridine, air, recycled exhaust gas and ammonia, was passed through the catalyst at a temperature of 345°C. The feeding rate (gram per 1 liter catalyst per hour = gl⁻¹h⁻¹) was: 3-methylpyridine 80 gl⁻¹h⁻¹, air 180 liter.h⁻¹, recycled exhaust gas 1200 liter.h⁻¹, ammonia 52.5 gl⁻¹h⁻¹ corresponding to a molar ratio of 3-methylpyridine to ammonia to oxygen of 1 : 3.6 : 2.0. Accordingly 1890 g 3-methylpyridine was fed over 24 hours. The conversion was 98.5%. 1850 g of 3-cyanopyridine were obtained corresponding to a yield of 88.5%. The output of 3-cyanopyridine was 77 gl⁻¹h⁻¹ .

### Example 10

A quantity of the obtained catalyst from example 8 (135 cm³, 160 g) was thermally treated at 620°C for 6 hours. This was loaded into a tube reactor made of stainless steel (internal diameter 21 mm, length 1000mm). A mixture of reagents, consisting of 3-methylpyridine, air, nitrogen and ammonia, was passed through the catalyst at a temperature of 375°C. The feeding rate was: 3-methylpyridine 11 gh⁻¹ (81 gl⁻¹h⁻¹ = gram per 1 liter catalyst per hour), air 30 liter.h⁻¹, nitrogen 285 liter.h⁻¹, ammonia 4 gh⁻¹ corresponding to a molar ratio of 3-methylpyridine to ammonia to oxygen of 1 : 2 : 2.6. Accordingly 264 g 3-methylpyridine was fed over 24 hours. The conversion was 99%. 261 g of 3-cyanopyridine were obtained corresponding to a yield of 89%. The output of 3-cyanopyridine was 80 gl⁻¹h⁻¹ .

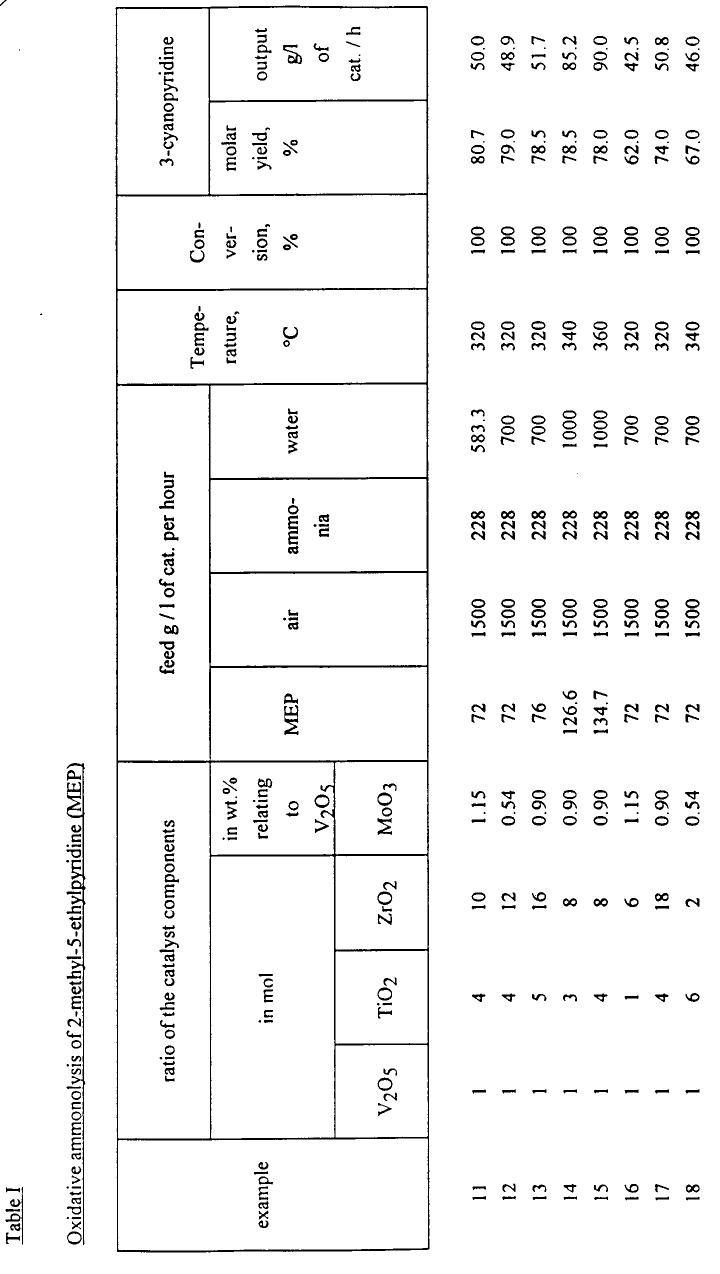

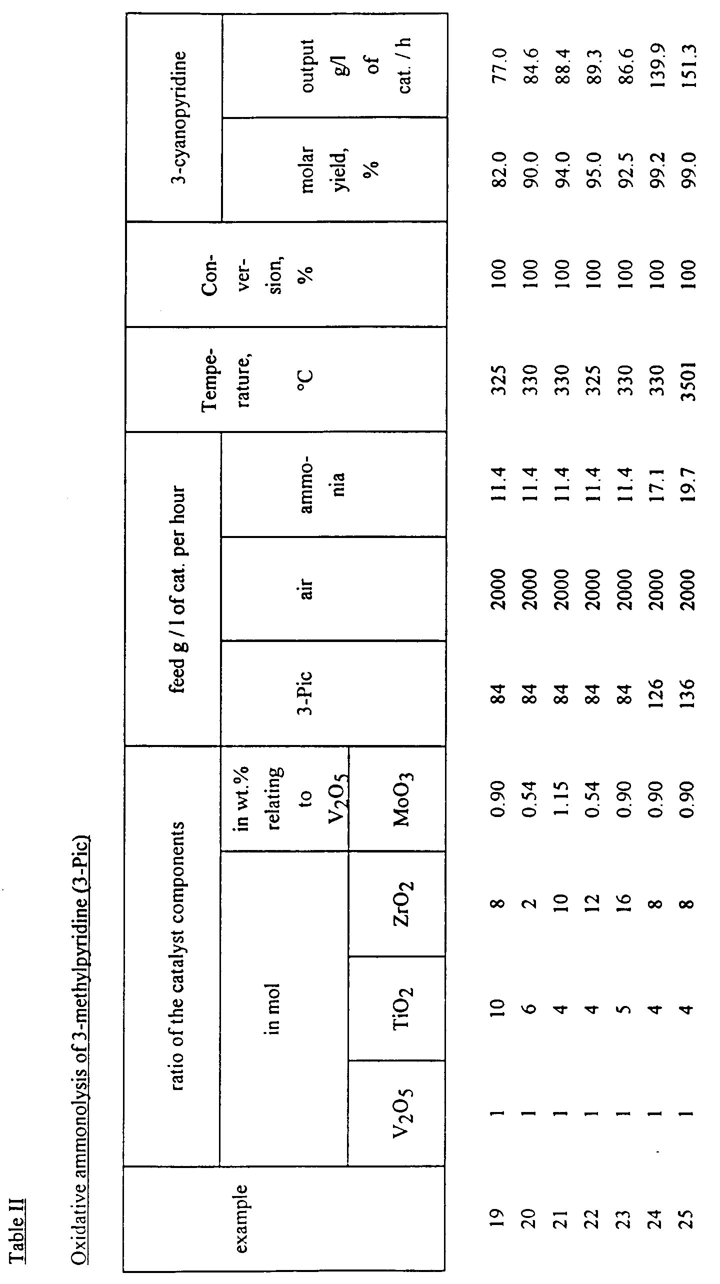

## Claims

1. Catalytic composition consisting of the oxides of vanadium, titanium, zirconium and molybdenum having a molar ratio of V₂O₅ to TiO₂ to ZrO₂ from 1:1:2 to 1:12:25 and having a MoO₃ content of 0.54 to 2.6 wt.% relating to V₂O₅.

2. Catalytic composition as claimed in claim 1 having a molar ratio of V₂O₅ to TiO₂ to ZrO₂ from 1:3:4 to 1:8:16 and having a MoO₃ content of 0.54 to 1.15 wt.% relating to V₂O₅.

3. Use of the catalytic compostion of claim 1 or 2 for the oxidative ammonolysis of alkylpyridines.

4. Use of the catalytic composition according to claim 3 for the oxidative ammonolysis of 3-methylpyridine and of 2-methyl-5-ethylpyridine.

5. Process for the preparation of cyanopyridines by oxidative ammonolysis of alkylpyridines characterised in that the alkylpyridine together with ammonia, an oxygen containing gas and if necessary water vapour is passed over the catalytic composition as claimed in claim 1 or 2 at a temperature from 280°C to 400°C.

6. Process according to claim 5 for the preparation of 3-cyanpyridine by oxidative ammonolysis of 3-methylpyridine characterised in that 3-methylpyridine, ammonia and an oxygen containing gas (calculated to O₂) in a molar ratio from 1:1:1.5 to 1:8.5:60 is passed over a catalytic composition as claimed in claim 1 or 2 at a temperature from 310°C to 380°C.

7. Process according to claim 6 characterised in that the molar ratio of 3-methylpyridine, ammonia, oxygen containing gas is 1:1:2 to 1:4:60.

8. Process according to claim 5 for the preparation of 3-cyanopyridine by oxidative ammonolysis of 2-methyl-5-ethylpyridine characterised in that 2-methyl-5-ethylpyridine, ammonia, an oxygen containing gas (calculated to O₂) and water vapour in a molar ratio from 1:20:20:60 to 1:60:70:330 is passed over a catalytic composition as claimed in claim 1 or 2 at a temperature from 310°C to 380°C.

## Patentansprüche

1. Katalytische Zusammensetzung, bestehend aus den Oxiden von Vanadium, Titan, Zirkonium und Molybdän mit einem Molverhältnis von V₂O₅ zu TiO₂ zu ZrO₂ von 1:1:2 bis 1:12:25 und mit einem MoO₃-Gehalt von 0,54 bis 2,6 Gew.-% in Bezug auf V₂O₅.

2. Katalytische Zusammensetzung nach Anspruch 1 mit einem Molverhältnis von V₂O₅ zu TiO₂ zu ZrO₂ von 1:3:4 bis 1:8:16 und mit einem MoO₃-Gehalt von 0,54 bis 1,15 Gew.-% in Bezug auf V₂O₅.

3. Verwendung der katalytischen Zusammensetzung gemäß Anspruch 1 oder 2 für die oxidative Ammonolyse von Alkylpyridinen.

4. Verwendung der katalytischen Zusammensetzung gemäß Anspruch 3 für die oxidative Ammonolyse von 3-Methylpyridin und von 2-Methyl-5-ethylpyridin.

5. Verfahren zur Herstellung von Cyanopyridinen durch oxidative Ammonolyse von Alkylpyridinen, dadurch gekennzeichnet, daß das Alkylpyridin zusammen mit Ammoniak, einem Sauerstoff enthaltenden Gas und falls notwendig Wasserdampf bei einer Temperatur von 280°C bis 400°C über die wie in Anspruch 1 oder 2 beanspruchte katalytische Zusammensetzung geleitet wird.

6. Verfahren nach Anspruch 5 zur Herstellung von 3-Cyanopyridin durch oxidative Ammonolyse von 3-Methylpyridin, dadurch gekennzeichnet, daß 3-Methylpyridin, Ammoniak und ein Sauerstoff enthaltendes Gas (berechnet als O₂) in einem Molverhältnis von 1:1:1,5 bis 1:8,5:60 bei einer Temperatur von 310°C bis 380°C über eine wie in Anspruch 1 oder 2 beanspruchte katalytische Zusammensetzung geleitet wird.

7. Verfahren nach Anspruch 6 dadurch gekennzeichnet, daß das Molverhältnis von 3-Methylpyridin, Ammoniak, Sauerstoff enthaltendem Gas gleich 1:1:2 bis 1:4:60 ist.

8. Verfahren nach Anspruch 5 zur Herstellung von 3-Cyanopyridin durch oxidative Ammonolyse von 2-Methyl-5-ethylpyridin, dadurch gekennzeichnet, daß 2-Methyl-5-ethylpyridin, Ammoniak, ein Sauerstoff enthaltendes Gas (berechnet als O₂) und Wasserdampf in einem Molverhältnis von 1:20:20:60 bis 1:60:70:330 bei einer Temperatur von 310°C bis 380°C über eine wie in Anspruch 1 oder 2 beanspruchte katalytische Zusammensetzung geleitet wird.

## Revendications

1. Composition catalytique constituée par des oxydes de vanadium, titane, zirconium et molybdène ayant un rapport molaire de V₂O₅ entre TiO₂ et ZrO₂ de 1 :1 :2 à 1 :12 :25 ayant une teneur MoO₃ de 0,54 à 2,6 % en poids par rapport à V₂O₅.

2. Composition catalytique selon la revendication 1 ayant un rapport molaire de V₂O₅ entre TiO₂ et ZrO₂ de 1 :3 :4 à 1 :8 :16 et ayant une teneur en MoO₃ de 0,54 à 1,15 % en poids rapporté à V₂O₅.

3. Utilisation de la composition catalytique de la revendication 1 ou 2 pour l'ammonolyse oxydative d'alkylpyridines.

4. Utilisation de la composition catalytique selon la revendication 3, pour l'ammonolyse oxydative de 3-méthlylpyridine et de 2-méthyl-5-éthylpyridine.

5. Procédé pour la préparation de cyanopyridines par ammonolyse oxydative d'alkylpyridines, caractérisé en ce que l'alkylpyridine conjointement avec l'ammoniac, un gaz contenant de l'oxygène et au besoin de la vapeur d'eau sont passés sur la composition catalytique selon la revendication 1 ou 2 à une température de 280°C à 400°C.

6. Procédé selon la revendication 5 pour la préparation de 3-cyanopyridine par ammonolyses oxydative de 3-méthylpyridine caractérisé en ce que la 3-méthylpyridine, l'ammoniac et un gaz contenant de l'oxygène (calculé par rapport à O₂) dans un rapport molaire de 1 :1 :1,5 à 1 :8,5 :60 sont passés sur une composition catalytique selon la revendication 1 ou 2 à une température de 310°C à 380°C.

7. Procédé selon la revendication 6 caractérisé en ce que le rapport molaire de 3-méthylpyridine, ammoniac, gaz contenant l'oxygène est de 1 :1 :2 à 1 :4 :60.

8. Procédé selon la revendication 5 pour la préparation de 3-cyanopyridine par ammonolyse oxydative de 2-méthyl-5-éthylpyridine caractérisé en ce que la 2-méthyl-5-éthylpyridine, ammoniac et un gaz contenant de l'oxygène (calculé sur O₂) et de la vapeur d'eau dans un rapport molaire de 1 :20 :20 :60 à 1 :60 :70 :330 sont passés sur une composition catalytique selon la revendication 1 ou 2 à une température de 310°C à 380°C.
